# EUROPEAN PATENT APPLICATION

(11) **EP 2 156 859 A2**
(43) Date of publication of application: **24.02.2010**
(21) Application number: 09166915.0
(22) Date of filing: 31.07.2009
(51) Int. Cl.: A61M 16/01, A61M 16/00, A61M 16/22

(54) **Automatic ventilator system and method**

(30) Priority: 18.08.2008 US 193427
(71) Applicant: General Electric Company, Schenectady, NY 12345 (US)
(72) Inventor: MASHAK, James Nyal, Sun Prairie, WI 53590 (US)
(74) Representative: Illingworth-Law, William Illingworth

(57) **Abstract**

A ventilator system (10) is disclosed herein. The ventilator system (10) includes a collapsible reservoir assembly (18) comprising an outer reservoir (26) and an inner reservoir (24) disposed at least partially within the outer reservoir (26). The ventilator system (10) also includes a source of gas (12) pneumatically coupled with the outer reservoir (26), and a controller (16) operatively connected to the source of gas (12). The controller (16) is configured to regulate the transmission of gas from the source of gas (12) to the outer reservoir (26) in order to compress the inner reservoir (24) and thereby automatically transfer the contents of the inner reservoir (24).

## Description

### FIELD OF THE INVENTION

This disclosure relates generally to an automatic ventilator system and method.

### BACKGROUND OF THE INVENTION

In general, medical ventilator systems are used to provide respiratory support to patients undergoing anesthesia and respiratory treatment whenever the patient's ability to breath is compromised. The primary function of the medical ventilator system is to maintain suitable pressure and flow of gases inspired and expired by the patient. Medical ventilator systems used in conjunction with anesthesia generally include an automatic system comprising a bellows and a manual system comprising a collapsible reservoir configured to allow a clinician to deliver manual breaths to the patient.

The manual system is implemented to ventilate a patient by repeatedly compressing and releasing the collapsible reservoir. When the collapsible reservoir is compressed, inhalation gas is transferred to the patient. When the collapsible reservoir is subsequently released, the patient passively exhales due to the lungs' elasticity. Fresh gas is generally continuously introduced into the system, and at least a portion of the patient's exhaled gas can be recycled and transferred back to the patient. A pressure release valve is traditionally provided to limit the pressure level in the manual system and thereby regulate the volume of inhalation gas transferred to the patient during each compression of the collapsible reservoir.

One problem with conventional medical ventilator systems relates to the expense associated with two separate sub-systems (i.e., an automatic system comprising a bellows and a manual system). Another problem relates to the packaging constraints imposed by the two separate sub-systems.

### BRIEF DESCRIPTION OF THE INVENTION

The above-mentioned shortcomings, disadvantages and problems are addressed herein which will be understood by reading and understanding the following specification.

In an embodiment, a ventilator system includes a collapsible reservoir assembly comprising an outer reservoir and an inner reservoir disposed at least partially within the outer reservoir. The ventilator system also includes a source of gas pneumatically coupled with the outer reservoir, and a controller operatively connected to the source of gas. The controller is configured to regulate the transmission of gas from the source of gas to the outer reservoir in order to compress the inner reservoir and thereby automatically transfer the contents of the inner reservoir.

In another embodiment, a ventilator system includes a collapsible reservoir assembly comprising an outer reservoir and an inner reservoir disposed at least partially within the outer reservoir. The inner reservoir being pneumatically connectable to a patient, and adapted to retain a patient gas. The ventilator system also includes a source of drive gas pneumatically coupled with the outer reservoir, and a controller operatively connected to the source of drive gas. The controller is configured to regulate the transmission of a drive gas from the source of drive gas to the outer reservoir in order to compress the inner reservoir and thereby automatically transfer the patient gas to the patient.

In another embodiment, a method includes providing an outer reservoir and an inner reservoir disposed at least partially within the outer reservoir, providing a source of drive gas pneumatically coupled with the outer reservoir. The method also include transmitting a selectable volume of drive gas from the source of drive gas to the outer reservoir in order to compress the inner reservoir and thereby automatically transfer the contents of the inner reservoir.

Various other features, objects, and advantages of the invention will be made apparent to those skilled in the art from the accompanying drawings and detailed description thereof.

### BRIEF DESCRIPTION OF THE DRAWINGS

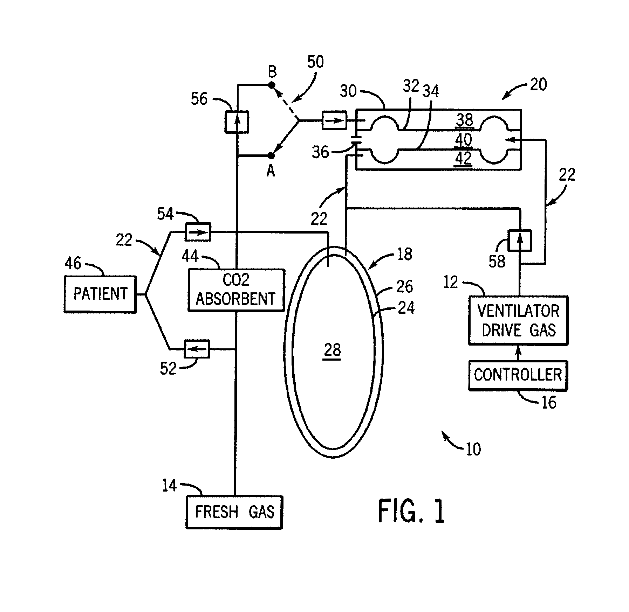

FIGURE 1 is a schematic representation of a ventilator system in accordance with an embodiment.

### DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

Referring to Figure 1, a medical ventilator system 10 is schematically depicted in accordance with an embodiment. The medical ventilator system 10 includes a source of ventilator drive gas 12, a source of fresh gas 14, a controller 16, a collapsible reservoir assembly 18, a diaphragm 20, and a pneumatic circuit 22. The pneumatic circuit 22 comprises a plurality of valves 50-58 and a plurality of hoses or tubes described in detail hereinafter.

The source of ventilator drive gas 12 and the source of fresh gas 14 may, for example, comprise a pressurized storage tank or a pump. The source of fresh gas 14 is configured to generally continuously introduce fresh gas into the pneumatic circuit 22 at a selectable rate. The source of ventilator drive gas 12 is regulated by the controller 16 in a known manner. According to one embodiment, the controller 16 may implement feedback from one or more pressure sensors (not shown) to regulate the source of ventilator drive gas 12 in order to maintain a selectable pressure level within the pneumatic circuit 22. According to another embodiment, the controller 16 may regulate the rate and/or duration during which ventilator drive gas is transmitted in order to deliver a selectable volume of gas to the pneumatic circuit 22.

The collapsible reservoir assembly 18 generally includes an inner reservoir 24 adapted to retain patient gas 28, and an outer reservoir 26. The inner and outer reservoirs 24, 26 comprise pliable bladders or bags that can be compressed and released in order to ventilate a patient. The inner reservoir 24 is disposed within the outer reservoir 26 such that a user can generally simultaneously compress both reservoirs 24, 26 with a single squeeze. The collapsible reservoir assembly 18 is commonly referred to as a hand bag, and is so named because an operator can use his or her hand to compress and release the reservoirs 24, 26. According to one embodiment, the outer reservoir 26 comprises a generally translucent material such that an operator can visually confirm inner reservoir 24 compression while squeezing the collapsible reservoir assembly 18. According to another embodiment, the outer reservoir 26 comprises a reinforced nylon construction rendering it compressible but resistant to stretching.

The diaphragm 20 comprises a housing 30, a first diaphragm seal 32, a second diaphragm seal 34, and a bleed orifice 36. The first and second diaphragm seals 32, 34 define first, second and third diaphragm chambers 38-42, respectively. The first and third chambers 38, 42 are adapted to release gas to atmosphere. According to one embodiment the first chamber 38 is adapted to release gas outside, and the third chamber 42 is adapted to release gas within the room. The introduction of gas into the second diaphragm chamber 40 causes the first and second diaphragm seals 32, 34 to expand or translate in an outward direction until the diaphragm seals 32, 34 engage an interior surface of the housing 30. When the diaphragm seals 32, 34 engage the interior surface of the housing 30, the first and third chambers 38, 42 become occluded such that gas cannot pass therethrough.

The manual ventilation operation mode of the medical ventilator system 10 will now be described in detail. When operating in manual ventilation mode, switch valve 50 is in position B identified with a dashed line, which has the effect of occluding position A.

When an operator generally simultaneously squeezes the inner and outer reservoirs 24, 26 of the collapsible reservoir assembly 18, at least a portion of the patient gas 28 in the inner reservoir 24 is transferred through a carbon dioxide (CO₂) absorbent 44, through the one-way valve 52, and to the patient 46. The CO₂ absorbent 44 is configured to scrub or remove any CO₂ from patient gas 28 in a known manner. When the collapsible reservoir assembly 18 is subsequently released the patient 46 passively exhales due to the elasticity of his or her lungs. The exhaled gas from the patient's lungs is transferred through the one-way valve 54, and is then combined with fresh gas from the source of fresh gas 14 before being transferred back to the inner reservoir 24.

According to one embodiment, an automatic pressure limiting (APL) valve 56 is provided. The APL valve 56 is configured to open when a predetermined pressure level is reached such that excess gas within the pneumatic circuit 22 can be transferred through switch valve 50 and vented to atmosphere via diaphragm chamber 38. The APL valve 56 can therefore be implemented to automatically limit the pressure level within the pneumatic circuit 22 regardless of variables such as the size of the inner reservoir 24 or the degree to which the inner reservoir 24 is compressed.

The automatic ventilation operation mode of the medical ventilator system 10 will now be described in detail. When operating in automatic ventilation mode, switch valve 50 is in position A identified with a solid line, which has the effect of occluding position B.

During automatic ventilation, the controller 16 regulates the source of ventilator drive gas 12 in order to transmit a selectable amount of drive gas into the pneumatic circuit 22. When drive gas is initially transmitted into the pneumatic circuit 22, the valve 58 prevents the drive gas from reaching the outer reservoir 26 such that the entire volume of drive gas is directed to the second diaphragm chamber 40. According to one embodiment, the valve 58 is configured to remain closed until the pressure level within the diaphragm chamber 40 reaches an amount necessary to expand the diaphragm seals 32, 34 into engagement with the housing 30 and thereby completely occlude diaphragm chambers 38, 42.

After the pressure level within the diaphragm chamber 40 reaches an amount necessary to occlude diaphragm chambers 38, 42, the valve 58 opens and drive gas is transferable therethrough to the outer reservoir 26. The drive gas introduced into the outer reservoir 26 has the effect of compressing the inner reservoir 24. Providing an outer reservoir 26 that is designed to minimize expansion reduces the volume of drive gas required to compress the inner reservoir 24, and also maintains consistency pertaining to drive gas volume versus degree of inner reservoir 24 compression. Implementing the controller 16 to transfer drive gas into the outer reservoir 26 causes the inner reservoir 24 to compress in an automatic manner, and thereby also transfers the patient gas 28 to the patient 46 as previously described with respect to the manual ventilation of a patient.

After automatically compressing the inner reservoir 24 to transfer patient gas 28 to the patient, the controller 16 shuts off the source of ventilator drive gas 12 such that drive gas is no longer introduced into the pneumatic circuit 22. When the source of ventilator drive gas 12 is shut off, the drive gas within the diaphragm chamber 40 is released through the bleed orifice 36. As drive gas is released through the bleed orifice 36, the pressure level with in the diaphragm chamber 40 is reduced thereby allowing the diaphragm seals 32, 34 to return to their steady state position and correspondingly opening diaphragm chambers 38, 42. When diaphragm chamber 42 is opened the drive gas within the outer reservoir 26 is released to atmosphere, which has the effect of releasing the inner reservoir 24 from its compressed state. When the inner reservoir 24 is released from its compressed state, the patient 46 can passively exhale due to the elasticity of his or her lungs. The exhaled gas from the patient's lungs is transferred through the one-way valve 54, and is then combined with fresh gas from the source of fresh gas 14 before being transferred back to the inner reservoir 24.

According to another embodiment, after automatically compressing the inner reservoir 24 to transfer patient gas 28 to the patient, the controller 16 may reduce drive gas flow rate instead of completely shutting off the source of ventilator drive gas 12. This has the effect of only partially releasing the drive gas within the outer reservoir 26 to atmosphere such that the inner reservoir 24 is correspondingly only partially released from its compressed state. This embodiment can be implemented to maintain positive end-expiratory pressure (PEEP) within the patient's airway at the end of the expiratory cycle.

By transmitting a predetermined volume of gas from the source of ventilator drive gas 12 to the outer reservoir 26, and by generally continuously transmitting the predetermined volume of gas at selectable intervals, the inner reservoir 24 can be periodically compressed and released in a manner adapted to automatically ventilate the patient 46. Advantageously, the medical ventilator system 10 does not incur the cost or packaging constraints associated with a bellows device that is generally required by conventional automatic ventilation systems. Additionally, the ventilator system 10 enables a user to squeeze the inner and outer reservoirs 24, 26 of the collapsible reservoir assembly 18 during automatic ventilation in order to manually supplement the transmission of patient gas 28.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

Aspects of the present invention are defined in the following numbered clauses:
1. A ventilator system comprising:
   a collapsible reservoir assembly comprising:
   an outer reservoir; and
   an inner reservoir disposed at least partially within the outer reservoir;
   a source of gas pneumatically coupled with the outer reservoir; and
   a controller operatively connected to the source of gas, wherein the controller is configured to regulate the transmission of a gas from the source of gas to the outer reservoir in order to compress the inner reservoir and thereby automatically transfer the contents of the inner reservoir.
2. The ventilator system of clause 1, wherein the controller is configured to regulate the transmission of the gas in order to compress the inner reservoir and thereby automatically ventilate a patient.
3. The ventilator system of clause 1, wherein the outer reservoir is generally translucent.
4. The ventilator system of clause 1, wherein the outer reservoir comprises a reinforced nylon material adapted to resist expansion.
5. The ventilator system of clause 1, wherein the source of gas comprises a pump.
6. The ventilator system of clause 1, wherein the source of gas comprises a pressurized storage tank.
7. The ventilator system of clause 1, further comprising a diaphragm pneumatically coupled with the outer reservoir and the source of gas.
8. The ventilator system of clause 1, further comprising a carbon dioxide absorbent pneumatically coupled with the inner reservoir.
9. A ventilator system comprising:
   a collapsible reservoir assembly comprising:
   an outer reservoir; and
   an inner reservoir disposed at least partially within the outer reservoir, said inner reservoir pneumatically connectable to a patient, said inner reservoir adapted to retain a patient gas;
   a source of drive gas pneumatically coupled with the outer reservoir; and
   a controller operatively connected to the source of drive gas, wherein the controller is configured to regulate the transmission of a drive gas from the source of drive gas to the outer reservoir in order to compress the inner reservoir and thereby automatically transfer the patient gas to the patient.
10. The ventilator system of clause 9, wherein the outer reservoir is generally translucent.
11. The ventilator system of clause 9, wherein the outer reservoir comprises a reinforced nylon material adapted to resist expansion.
12. The ventilator system of clause 9, further comprising a diaphragm pneumatically coupled with the outer reservoir and the source of gas.
13. The ventilator system of clause 9, further comprising a carbon dioxide absorbent pneumatically coupled with the inner reservoir.
14. A method comprising;
   providing an outer reservoir and an inner reservoir disposed at least partially within the outer reservoir;
   providing a source of drive gas pneumatically coupled with the outer reservoir; and
   transmitting a selectable volume of drive gas from the source of drive gas to the outer reservoir in order to compress the inner reservoir and thereby automatically transfer the contents of the inner reservoir.
15. The method of clause 14, wherein said providing an outer reservoir comprises providing a generally translucent outer reservoir.
16. The method of clause 14, wherein said providing an outer reservoir comprises providing an outer reservoir composed of a reinforced nylon material adapted to resist expansion.
17. The method of clause 14, wherein said providing a source of drive gas comprises providing a pump.
18. The method of clause 14, wherein said providing a source of drive gas comprises providing a pressurized tank.

## Claims

1. A ventilator system (10) comprising:
a collapsible reservoir assembly (18) comprising:
an outer reservoir (26); and
an inner reservoir (24) disposed at least partially within the outer reservoir (26);
a source of gas (12) pneumatically coupled with the outer reservoir (26); and
a controller (16) operatively connected to the source of gas (12), wherein the controller (16) is configured to regulate the transmission of a gas from the source of gas (12) to the outer reservoir (26) in order to compress the inner reservoir (24) and thereby automatically transfer the contents of the inner reservoir (24).

2. The ventilator system (10) of claim 1, wherein the controller (16) is configured to regulate the transmission of the gas in order to compress the inner reservoir (24) and thereby automatically ventilate a patient (46).

3. The ventilator system (10) of claim 1 or claim 2, wherein the outer reservoir (26) is generally translucent.

4. The ventilator system (10) of any one of the preceding claims, wherein the outer reservoir (26) comprises a reinforced nylon material adapted to resist expansion.

5. The ventilator system (10) of any one of the preceding claims, wherein the source of gas (12) comprises a pump.

6. The ventilator system (10) of any one of the preceding claims, wherein the source of gas (12) comprises a pressurized storage tank.

7. The ventilator system (10) of any one of the preceding claims, further comprising a diaphragm (20) pneumatically coupled with the outer reservoir (26) and the source of gas (12).

8. The ventilator system (10) of any one of the preceding claims, further comprising a carbon dioxide absorbent (44) pneumatically coupled with the inner reservoir (24).

9. A method comprising;
providing an outer reservoir (26) and an inner reservoir (24) disposed at least partially within the outer reservoir (26);
providing a source of drive gas (12) pneumatically coupled with the outer reservoir (26); and
transmitting a selectable volume of drive gas from the source of drive gas (12) to the outer reservoir (26) in order to compress the inner reservoir (24) and thereby automatically transfer the contents of the inner reservoir (24).

10. The method of claim 9, wherein said providing an outer reservoir (26) comprises providing a generally translucent outer reservoir.

11. The method of claim 9 or claim 10, wherein said providing an outer reservoir (26) comprises providing an outer reservoir composed of a reinforced nylon material adapted to resist expansion.

12. The method of any one of claims 9 to 11, wherein said providing a source of drive gas (12) comprises providing a pump.

13. The method of any one of claims 9 to 12, wherein said providing a source of drive gas (12) comprises providing a pressurized tank.
